# FASCICULE DE BREVET EUROPEEN

(11) **EP 0 646 371 B1**
(45) Date de publication et mention de la délivrance du brevet: **07.07.1999**
(21) Numéro de dépôt: 94402210.2
(22) Date de dépôt: 04.10.1994
(51) Int. Cl.: A61K 7/48, A61K 31/07

(54) **Composition cosmétique ou pharmaceutique, anhydre et à base de rétinol**
Wasserfreies kosmetisches oder pharmazeutisches Mittel enthaltend Retinol
Anhydrous cosmetic or pharmaceutical composition containing retinol

(30) Priorité: 05.10.1993 FR 9311850
(43) Date de publication de la demande: 05.04.1995
(73) Titulaire: L'OREAL, 75008 Paris (FR)
(72) Inventeur: Segot, Evelyne, F-94130 Nogent sur Marne (FR); Laugier, Jean-Pierre, F-92160 Antony (FR)
(74) Mandataire: Stalla-Bourdillon, Bernard

(56) Documents cités:
- EP-A- 0 129 003
- DE-A- 1 935 939
- FR-A- 2 515 034
- GB-A- 1 126 289
- US-A- 3 584 115
- US-A- 4 022 913
- S.T.N., File Supplier, KARLSRUHE,DE, File Chemical Abstracts, vol 85, n 83131 * résumé * & JP-A-51012591 (KANEBO)

## Description

La présente invention a pour objet une composition cosmétique ou pharmaceutique pour la peau, anhydre et stable, à base de rétinol ou vitamine A et son utilisation en vue du traitement des maladies de la peau, en particulier du traitement de l'acné, des troubles de la kératinisation ou de la cicatrisation, du photovieillissement ou de la prévention et de l'atténuation des rides.

Le rétinol a déjà été préconisé dans ce domaine de traitement par exemple dans la demande de brevet européen n° 224.504 qui décrit une composition stable comprenant du rétinol, et une silicone volatile ainsi qu'un solvant du rétinol et de la silicone volatile.

Le solvant utilisé est de préférence l'éthanol, mais des études effectuées sur ce type de composition ont montré que le rétinol se dégradait en présence d'éthanol.

Une composition cosmétique anhydre contenant comme principe actif topique, du rétinol a également été décrite dans la demande de brevet européen n° 255.364. Cette composition comprend, outre le rétinol, un émollient liquide, un agent épaississant et un agent solubilisant ainsi que des agents antioxydants et autres conservateurs.

Il a également été décrit dans le brevet US 3,584,115 des compositions aérosols à base d'alcools gras linéaires pouvant contenir diverses vitamines dont la vitamine A.

On a maintenant découvert de manière inattendue et surprenante que par une sélection bien déterminée de certains solvants non miscibles à l'eau, comme véhicule du rétinol, on pouvait obtenir des compositions anhydres destinées à une application topique présentant une grande stabilité dans le temps et ceci sans avoir recours à la présence d'agents antioxydants.

La présente invention a donc pour objet une composition cosmétique ou pharmaceutique pour la peau, anhydre et stable, à base de rétinol, caractérisée par le fait qu'elle contient le rétinol à l'état solubilisé dans un solvant organique liquide à température ambiante choisi dans le groupe constitué par :
i) les alcools gras aliphatiques à chaîne ramifiée en C₁₆-C₂₀,
ii) les alcools gras saturés à chaîne droite ou ramifiée en C₁₆-C₂₀ alcoxylés,
iii) les diesters d'acides dicarboxyliques en C₆-C₁₄ et d'alcool isopropylique, et les mélanges desdits solvants.

Grâce à cette sélection particulière de solvants, la composition selon l'invention est particulièrement stable dans le temps aucune dégradation significative du rétinol n'étant observée même en l'absence de tout adjuvant en particulier d'agents antioxydants.

Les essais de conservation réalisés ont en effet permis de montrer que par rapport à d'autres solvants couramment utilisés comme véhicule de substance active, ceux sélectionnés selon la présente invention conduisaient à une nette amélioration de la stabilité.

Les alcools gras aliphatiques à chaîne ramifiée en C₁₆-C₂₀ utilisables selon l'invention sont de préférence saturés et parmi ceux-ci, on peut citer l'alcool isostéarylique tel que celui vendu par la Société Sherex sous la dénomination de "Adol 66", l'hexyl-2 décanol tel que celui vendu par la Société Condea sous la dénomination de "Isofol 16" et l'octyl dodécanol tel que celui vendu par la Société Henkel sous la dénomination de "Eutanol G".

Parmi les alcools gras saturés à chaîne droite ou ramifiée en C₁₆-C₂₀ alcoxylés utilisables selon l'invention, on préfère utiliser ceux comportant de 10 à 50 moles d'oxyde d'éthylène et/ou d'oxyde de propylène.

Parmi ceux-ci, on peut citer notamment l'alcool stéarylique oxypropyléné à l'aide de 15 moles d'oxyde de propylène tel que celui vendu par la Société ICI sous la dénomination de "Arlamol E".

Parmi les diesters d'acides dicarboxyliques en C₆-C₁₄ et d'alcool isopropylique utilisables selon l'invention, on peut citer l'adipate de di-isopropyle tel que celui vendu par la Société ISP sous la dénomination de "Ceraphyl 230".

La composition selon l'invention telle que définie ci-dessus, peut être utilisée telle quelle dans le traitement local des maladies de la peau ainsi que dans les traitements anti-vieillissement.

De manière générale, le rétinol est présent dans la composition selon l'invention en une proportion comprise entre 0,001 et 15 % et de préférence entre 0,01 et 10 % en poids par rapport au poids total de la composition, le reste étant essentiellement constitué par le solvant organique liquide à température ambiante tel que défini ci-dessus.

Certains ingrédients usuels peuvent être introduits dans la composition selon l'invention, comme ceci est conventionnel pour les compositions cosmétiques ou pharmaceutiques anhydres dans la mesure où ceux-ci sont solubles et n'en modifient pas la stabilité.

Toutefois, selon un mode de réalisation préféré, la composition selon l'invention, se présente sous une forme destinée à une application topique telle que par exemple une lotion constituée uniquement du rétinol en solution dans au moins un des solvants organiques précédemment définis.

La présente invention a également pour objet une composition conditionnée en deux parties, la première partie étant constituée par la solution de rétinol dans un des solvants organiques mentionnés ci-dessus et la deuxième partie étant constituée d'un support cosmétique ou pharmaceutique conventionnel sous forme, par exemple d'un gel, d'une émulsion, d'une pommade, les deux parties étant mélangées au moment de l'emploi et conduisant à la formation d'un lait.

Le support cosmétique ou pharmaceutique outre la présence d'un agent gélifiant ou d'un agent émulsionnant peut en outre contenir divers additifs tels que des filtres, des parfums des substances colorantes ou des charges.

### ETUDE DE STABILITE

Cette étude a eu pour but d'examiner la stabilité du rétinol en association avec différents solvants au cours du temps et en fonction de la température.

Connaissant la sensibilité du rétinol à l'air, à la lumière et à la chaleur, on a respecté certaines conditions de préparation des échantillons. Ainsi toute manipulation a été effectuée dans une pièce occultée en lumière inactinique et sous azote. Les divers solvants ont été préalablement désoxygénés par barbotage d'azote puis le rétinol a été solubilisé par agitation magnétique à température ambiante. Pour chaque solvant, 2 compositions ont été testées à des rapports pondéraux rétinol/solvant de 10 %. Les échantillons ont ensuite été conditionnés sous azote dans des flacons en verre brun, protégés de la lumière. Ceux-ci ont ensuite été conservés à 4°C, à la température ambiante (TA), à 37°C ainsi qu'à 45°C pendant deux mois.

Après ce temps, les dosages ont été réalisés selon la technique de chromatographie liquide haute performance (HPLC) dans les conditions suivantes :
Colonne : 2 colonnes 15 cm - diamètre 4 mm
Spherisorb ODS1 5 µm
Alltech ODS1
Eluant : gradient de solvants

| | | | |
|---|---|---|---|
| Solvant A : | acétonitrile = 850 ml | | |
| | acétate d'ammonium anhydre = 400 mg | | |
| | acide acétique à 100 % = 10 ml | | |
| | eau = 140 ml | | |
| Solvant B : | acétonitrile = 60 ml | | |
| | isopropanol = 40 ml | | |

| Gradient : | Temps (mn) | A (%) | B (%) |
|---|---|---|---|
| | 0 | 100 | - |
| | 20 | 100 | - |
| | 25 | - | 100 |
| | 35 | - | 100 |
| | 40 | 100 | - |
| | 50 | 100 | - |

Débit : 1 ml/mn
Détection : Spectrophotométrie UV à 340 nm
Gamme d'étalonnage et échantillon : Préparation d'une gamme de solutions éthanoliques de rétinol de 25 à 100 µg/ml - Injection 10 µl
Préparation des échantillons = dilution dans l'éthanol

Les résultats, correspondant à la moyenne de 3 mesures, sont exprimés en termes de pourcentage de rétinol dégradé, après deux mois de stockage dans les conditions indiquées ci-dessus. Les valeurs obtenues pour chaque solvant sont données dans le tableau (1) ci-dessous.

**TABLEAU 1**

| Températures | +4°C | TA | 37°C | 45°C |
|---|---|---|---|---|
| Propylène glycol | -25 | -34 | -65 | -84 |
| Ethanol | 0 | -3 | -6 | -21 |
| Alcool stéarylique oxypropyléné à 15 moles d'oxyde de propylène | 0 | 0 | 0 | -9 |
| Alcool isostéarylique | 0 | 0 | 0 | -8 |
| Hexyl-2 décanol | 0 | 0 | 0 | 0 |
| Octyl dodécanol | 0 | 0 | 0 | 0 |
| Adipate de di-isopropyle | 0 | 0 | 0 | 0 |

On va maintenant donner à titre d'illustration plusieurs exemples de composition selon l'invention à base de rétinol. Celles-ci ont été préparées de la même manière que celles utilisées pour l'étude de stabilité décrite ci-dessus.

### EXEMPLES DE COMPOSITIONS

### Exemple 1: Lotion topique

| | |
|---|---|
| - alcool stéarylique oxypropyléné à l'aide de 15 moles d'oxyde de propylène | 90 % |
| - rétinol | 10 % |

### Exemple 2 : Lotion topique

| | |
|---|---|
| - octyl dodécanol | 90 % |
| - rétinol | 10 % |

### Exemple 3 : Lotion topique

| | |
|---|---|
| - hexyl-2 décanol | 91 % |
| - rétinol | 9 % |

Les lotions des exemples 1 à 3 ci-dessus appliquées une fois par semaine sur les parties de la peau à traiter permettent de prévenir et d'atténuer la formation des rides.

### Exemple 4 : Composition conditionnée en deux parties pour la préparation d'un lait.

Au moment de l'emploi, on mélange la Partie A suivante :

| Partie A : | |
|---|---|
| - adipate de di-isopropyle | 9,5 g |
| - rétinol | 0,5 g |

à la Partie B suivante :

| Partie B : | |
|---|---|
| - gomme de xanthane | 0,7 g |
| - conservateur | 0,3 g |
| - eau | 89,0 g |

On obtient un produit gélifié laiteux que l'on applique sur les parties acnéiques de la peau à traiter. Après une application deux fois par semaine pendant 4 à 5 semaines environ, on note une diminution des zones acnéiques.

Cette même composition appliquée sur une peau saine permet de prévenir et d'atténuer les rides.

## Revendications

1. Composition cosmétique ou pharmaceutique pour la peau, anhydre et stable, à base de rétinol, caractérisée par le fait qu'elle contient le rétinol à l'état solubilisé dans un solvant organique, liquide à température ambiante, choisi dans le groupe constitué par :
i) les alcools gras aliphatiques à chaîne ramifiée en C₁₆-C₂₀,
ii) les alcools gras saturés à chaîne droite ou ramifiée en C₁₆-C₂₀ alcoxylés,
iii) les diesters d'acides dicarboxyliques en C₆-C₁₄ et d'alcool isopropylique, et les mélanges desdits solvants.

2. Composition selon la revendication 1, caractérisée par le fait qu'elle est exempte de tout agent antioxydant.

3. Composition selon l'une quelconque des revendications précédentes, caractérisée par le fait que le rétinol est présent en une proportion comprise entre 0,001 et 15 % et de préférence entre 0,01 et 10 % en poids par rapport au poids total de la composition, le reste étant essentiellement constitué par le solvant organique.

4. Composition selon l'une quelconque des revendications précédentes, caractérisée par le fait que l'alcool gras aliphatique à chaîne ramifiée en C₁₆-C₂₀ est choisi parmi l'alcool isostéarylique, l'hexyl-2 décanol et l'octyl dodécanol.

5. Composition selon l'une quelconque des revendications 1 à 3, caractérisée par le fait que l'alcool gras saturé à chaîne droite ou ramifiée en C₁₆-C₂₀ alcoxylé est l'alcool stéarylique oxypropyléné à l'aide de 15 moles d'oxyde de propylène.

6. Composition selon l'une quelconque des revendications 1 à 3, caractérisée par le fait que le diester d'acide dicarboxylique en C₆-C₁₄ et d'alcool isopropylique est l'adipate de di-isopropyle.

7. Conditionnement en deux parties caractérisé par le fait que la première partie est constituée par une composition selon l'une quelconque des revendications 1 à 6 et que la deuxième partie est constituée d'un support cosmétique ou pharmaceutique conventionnel, les deux parties par mélange au moment de l'emploi conduisant à la formation d'un lait.

## Claims

1. Anhydrous, stable, retinol-based cosmetic or pharmaceutical composition for the skin, characterized in that it contains retinol in dissolved form in an organic solvent, which is liquid at room temperature, chosen from the group consisting of:
i) branched-chain C₁₆-C₂₀ aliphatic fatty alcohols,
ii) straight- or branched-chain alkoxylated C₁₆-C₂₀ saturated fatty alcohols,
iii) diesters of C₆-C₁₄ dicarboxylic acids and of isopropyl alcohol and mixtures of the said solvents.

2. Composition according to Claim 1, characterized in that it is free of any antioxidant.

3. Composition according to either of the preceding claims, characterized in that the retinol is present in a proportion of between 0.001 and 15% and preferably between 0.01 and 10% by weight relative to the total weight of the composition, the remainder consisting essentially of the organic solvent.

4. Composition according to any one of the preceding claims, characterized in that the branched-chain C₁₆-C₂₀ aliphatic fatty alcohol is chosen from isostearyl alcohol, 2-hexyldecanol and octyldodecanol.

5. Composition according to any one of Claims 1 to 3, characterized in that the straight- or branched-chain alkoxylated C₁₆-C₂₀ saturated fatty alcohol is stearyl alcohol oxypropylenated with 15 mol of propylene oxide.

6. Composition according to any one of Claims 1 to 3, characterized in that the diester of C₆-C₁₄ dicarboxylic acid and of isopropyl alcohol is diisopropyl adipate.

7. Packaging in two parts, characterized in that the first part consists of a composition according to any one of Claims 1 to 6 and in that the second part consists of a conventional cosmetic or pharmaceutical support, the two parts leading to the formation of a milk on mixing together at the time of use.

## Patentansprüche

1. Wasserfreie und stabile kosmetische oder pharmazeutische Zubereitung für die Haut auf Basis von Retinol, dadurch gekennzeichnet, daß sie das Retinol in solubilisiertem Zustand in einem organischen, bei Umgebungstemperatur flüssigen Lösungsmittel enthält, das gewählt ist aus der Gruppe, bestehend aus:
(i) aliphatischen C₁₆-C₂₀-Fettalkoholen mit verzweigter Kette,
(ii) alkoxylierten gesättigten C₁₆-C₂₀-Fettalkoholen mit gerader oder verzweigter Kette,
(iii) den Diestern von C₆-C₁₄-Dicarbonsäuren mit Isopropylalkohol sowie Mischungen dieser Lösungsmittel.

2. Zubereitung gemäß Anspruch 1, dadurch gekennzeichnet, daß sie frei von jeglichem Antioxidans ist.

3. Zubereitung gemäß einem der vorstehenden Ansprüche, dadurch gekennzeichnet, daß das Retinol in einer Menge zwischen 0,001 und 15 Gew.-% und vorzugsweise 0,01 und 10 Gew.-%, bezogen auf das Gesamtgewicht der Zubereitung, enthalten ist, wobei die Restmenge im wesentlichen aus dem organischem Lösungsmittel besteht.

4. Zubereitung gemäß einem der vorstehenden Ansprüche, dadurch gekennzeichnet, daß der aliphatische C₁₆-C₂₀-Fettalkohol mit verzweigter Kette unter Isostearylalkohol, 2-Hexyldecanol und Octyldodecanol ausgewählt ist.

5. Zubereitung gemaß einem der Ansprüche 1 bis 3, dadurch gekennzeichnet, daß der alkoxylierte gesättigte C₁₆-C₂₀-Fettalkohol mit gerader oder verzweigter Kette ein mit Hilfe von 15 Mol Propylenoxid propoxylierter Stearylalkohol ist.

6. Zubereitung gemäß einem der Ansprüche 1 bis 3, dadurch gekennzeichnet, daß der Diester der C₆-C₁₄-Dicarbonsäure mit Isopropylalkohol der Adipinsäurediisopropylester ist.

7. Aufmachung in zwei Teilen, dadurch gekennzeichnet daß der erste Teil aus einer Zubereitung gemaß einem der Ansprüche 1 bis 6 und der zweite Teil aus einem herkömmlichen kosmetischen oder pharmazeutischen Träger besteht, wobei beide Teile durch Mischen im Moment der Anwendung zur Bildung einer Milch führen.
